(19) Europäisches Patentamt / European Patent Office / Office européen des brevets

(11) **EP 2 691 521 B1**

(12) **EUROPEAN PATENT SPECIFICATION**

(45) Date of publication and mention
of the grant of the patent:
**04.11.2015 Bulletin 2015/45**

(51) Int Cl.:
*C12N 13/00* (2006.01)     *C12N 7/00* (2006.01)
*C12N 7/04* (2006.01)     *C12M 1/42* (2006.01)
*A23C 3/07* (2006.01)     *A23C 7/00* (2006.01)
*A23C 19/097* (2006.01)     *A23L 1/025* (2006.01)
*A23L 3/30* (2006.01)     *A61L 2/025* (2006.01)

(21) Application number: **12710982.5**

(22) Date of filing: **29.03.2012**

(86) International application number:
**PCT/EP2012/055739**

(87) International publication number:
**WO 2012/131008 (04.10.2012 Gazette 2012/40)**

(54) **INACTIVATION OF BACTERIOPHAGES IN A LIQUID**

INAKTIVIERUNG VON BAKTERIOPHAGEN IN EINER FLÜSSIGKEIT

INACTIVATION DE BACTÉRIOPHAGES DANS UN LIQUIDE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **29.03.2011 DK 201100231 P
20.01.2012 DK 201200054 P**

(43) Date of publication of application:
**05.02.2014 Bulletin 2014/06**

(73) Proprietor: **Chr. Hansen A/S
2970 Hørsholm (DK)**

(72) Inventor: **DUPONT, Kitt
DK-2990 Nivå (DK)**

(74) Representative: **UEXKÜLL & STOLBERG
Patentanwälte
Beselerstrasse 4
22607 Hamburg (DE)**

(56) References cited:
**WO-A1-2008/063115     DE-A1- 3 903 648
GB-A- 671 922**

• A. P. KRUEGER: "THE EFFECT OF SONIC
VIBRATIONS ON PHAGE, PHAGE PRECURSOR,
AND THE BACTERIAL SUBSTRATE", THE
JOURNAL OF GENERAL PHYSIOLOGY, vol. 24,
no. 6, 20 July 1941 (1941-07-20), pages 691-698,
XP055032163, ISSN: 0022-1295

• PINTO F ET AL: "Effect of surfactants,
temperature, and sonication on the virucidal
activity of polyhexamethylene biguanide against
the bacteriophage MS2", AMERICAN JOURNAL
OF INFECTION CONTROL, vol. 38, no. 5, 1 June
2010 (2010-06-01), pages 393-398, XP027088403,
ISSN: 0196-6553

• DATABASE BIOSIS [Online] BIOSCIENCES
INFORMATION SERVICE, PHILADELPHIA, PA,
US; 1975, DEBSKA W ET AL: "ULTRASOUND IN
PHARMACEUTICAL STUDIES PART 1",
XP002679555, Database accession no.
PREV197662033419 & DEBSKA W ET AL:
"ULTRASOUND IN PHARMACEUTICAL STUDIES
PART 1", HERBA POLONICA, vol. 21, no. 1, 1975,
pages 91-97, ISSN: 0018-0599

• DATABASE BIOSIS [Online] BIOSCIENCES
INFORMATION SERVICE, PHILADELPHIA, PA,
US; 2002, VAN DER WALT E: "The effect of
ultraviolet light, cavitational flow and ultrasound
on protozoan cysts and oocysts, bacteriophages
and Clostridium.", XP002679556, Database
accession no. PREV200300290930 & VAN DER
WALT E: "The effect of ultraviolet light,
cavitational flow and ultrasound on protozoan
cysts and oocysts, bacteriophages and
Clostridium.", WATER SA (PRETORIA), no.
Special Edition, 2002, pages 16-22, ISSN:
0378-4738

- ATAMER Z ET AL: "Thermal inactivation of the heat-resistant Lactococcus lactis bacteriophage P680 in modern cheese processing", INTERNATIONAL DAIRY JOURNAL, vol. 20, no. 3, 1 March 2010 (2010-03-01), pages 163-168, XP026833951, ISSN: 0958-6946

- P PIYASENA ET AL: "Inactivation of microbes using ultrasound: a review", INTERNATIONAL JOURNAL OF FOOD MICROBIOLOGY, vol. 87, no. 3, 1 November 2003 (2003-11-01), pages 207-216, XP055032155, ISSN: 0168-1605

**Description**

**FIELD OF INVENTION**

The present invention relates to inactivation of bacteriophages In a liquid, such as a dairy product, milk, milk solution, whey, must, and juice.

**BACKGROUND OF INVENTION**

Bacteriophages, also called phages, are viruses that attack bacteria. Phages are highly host specific, meaning that any given phage will attack a particular species or group of species of bacteria. Phage infections may be very destructive in bioprocesses for a company's productivity.

A broad number of food products, commodity chemicals, and biotechnology products are manufactured Industrially by large-scale bacterial fermentation of various organic substrates. Because enormous amounts of bacteria are being cultivated each day In large fermentation vats, the risk that bacteriophage contamination rapidly brings fermentations to a halt and cause economical setbacks is a serious threat in these industries.

Phages infecting lactic acid bacteria (LAB) are of considerable economic importance as they are the main cause of fermentation failure in the dairy Industry. Especially, phage attack is the cause of much loss to the cheese-making. Cheese Is made of milk with the aid of starter cultures containing lactic acid bacteria. The use of such starter cultures has been accompanied by the occasional failure of the starter culture in acidifying the milk in the cheese vat.

Significant technological advances in the industry have failed to alleviate this problem and consequently biological research aimed at understanding phage-host interactions as well as phage biology Is targeted at providing efficient solutions to the problem of phage infection.

GB 671 922 A describes that by treating products with oscillatory energy, microorganisms, in particular bacteria and viruses, can be destroyed. However, there is no experimental evidence in GB 671 922 A that could support this allegation and there Is no clear indication which microorganisms shall be inactivated by the oscillatory energy. DE 39 03 648 A1 discloses a method for the inactivation of viruses, such as the phage MAL 315 in an aqueous nutrient broth by treatment of the solution with ultrasonic sound. However, DE 39 03 648 A1 does not specifically disclose the inactivation of phages in a dairy product, milk, milk solution, whey, must or juice by sonication.

Modern methods for controlling phage problems In cheesemaking include:

- Use of starters containing phage-unrelated or phage-insensitive strains.
- Aseptic propagation systems
- Use of phage-inhibitory media
- Culture rotations
- Cleaning/chlorination of vats between refills

However, these methods are not sufficient for alleviating the phage problem.

Atamer et al. (2010, Intern Dairy J, 20(3): 163-168) pertains to a method for inactivating bacteriophages In whey and contemplates subjecting the whey to a heat treatment in order to inactivate common phages. It is indicated that alternative inactivation methods should be used e.g. for thermo-resistant phages. UV treatment is disclosed as the preferred alternative method for disabling such phages in whey. Ultrasound treatment of whey is not mentioned in this publication.

**SUMMARY OF INVENTION**

There is a need for alternative methods for combating phages in the dairy Industry. The present Inventor has surprisingly discovered that ultrasound can inactivate phages in milk and whey without considerably changing the flavor and functional quality of the liquid, and therefore It is commercially Interesting to use ultrasound for combating phages that Infects lactic acid bacteria.

Based on this surprising finding, the present Invention relates to a method that is useful for inactivation of bacteriophages In a liquid, comprising sonication (ultrasound treatment) of the liquid, wherein the liquid is selected from the group consisting of: a dairy product, milk, milk solution, whey, must, and juice. Also, the Invention relates to the use of a sonicator for inactivation of bacteriophages in a dairy product (such as cheese, cheese curd, fermented milk, milk, whey, whey permeate or cream), and to a dairy product obtained by the method of the Invention.

Further, the present invention relates to equipment useable In the method, ie apparatus for inactivation of bacteriophages in a liquid, wherein said apparatus is a dairy process line comprising a sonicator or a sonicator unit and a cheese vat.

**DETAILED DISCLOSURE**

In a first aspect, the present invention relates to a method for treatment of a liquid, which results in inactivation of bacteriophages present in the liquid, said method comprises sonication (ultrasound treatment) of the liquid, wherein the liquid is selected from the group consisting of: a dairy product, milk, milk solution, whey, must, and juice. A preferred embodiment of the first aspect Is a method for inactivation of bacteriophages in a liquid, comprising sonication of the liquid.

In the first aspect, it is preferred that the majority of the phages is inactivated. Preferably, at least 70%, at least 75%, at least 80%, at least 85%, or at least 90% of the phages in the liquid are Inactivated. In a particularly preferred aspect, at least 95%, at least 96%, at least 97%, at least 98%, at least 99%, at least 99.9%, or at least 99.99% of the phages that are present In the liquid are Inactivated. Inactivation of the phages can be determined, e.g. by spot test method described In Example 1.

The liquid to be treated by the method of the invention is selected from the group consisting of: a dairy product, milk, milk solution, whey, must, and juice. Especially interesting is a liquid selected from the group consisting of milk, milk solution and whey.

The sonication may be carried out by treatment of the liquid with ultrasound at a frequency in the range 10000 to 50000 Hz, such as in the range 13000 to 34000, 15000 to 25000 Hz or in the range 18000 to 22000 Hz.

It is presently preferred that the sonication is carried out by treatment of the liquid with ultrasound for more than 0.1 minutes, such as for more than 1.0, more than 1.2, more than 1.5, more than 2.0 or more than 2.5 minutes, and it is presently preferred that the sonication is carried out by treatment of the liquid with ultrasound for less than 30 minutes, such as for less than 15, less than 10, less than 9, less than 8, or less than 7 minutes.

Interesting embodiments of the method of the invention is embodiments wherein the sonication is carried out by treatment of the liquid with ultrasound for 0.1 to 30 minutes, such as for 1.0 to 15, 1.2 to 11, 1.5 to 9, 2.0 to 8, for 2.5 to 7, or for 3 to 6 minutes.

The sonication may be carried out by treatment of the liquid with ultrasound at an effect of more than 300 watt per liter liquid, such as more than 500, 900, 1100, 1300, 1500 or more than 1600 watt per liter liquid, but it is presently preferred that the sonication is carried out by treatment of the liquid with ultrasound at an effect of less than 6000 watt per liter liquid, such less than 5000, 4000, 3000, 2500, 2000, 1900 or less than 1800 watt per liter liquid.

In an interesting embodiment, the sonication is carried out by treatment of the liquid with ultrasound at an effect of 100 to 20000 watt per liter liquid, such as 200 to 15000, 300 to 10000, 400 to 5000, or 600 to 2250 watt per liter liquid, or the sonication is carried out by treatment of the liquid with ultrasound at an effect of 300 to 5000 watt per liter liquid, such as 600 to 4000, 900 to 3000, 1100 to 2500, or 1300 to 2000 watt per liter liquid.

In yet an interesting embodiment of the method of the invention, the sonication is carried out by treatment of the liquid with ultrasound at an effect of 1000 to 2000 watt per liter liquid for 1 to 10 minutes, such as for 3 to 7 minutes.

When the liquid to be treated is for human consumption, such as milk, the working temperature of the process is preferably kept at a level where the organoleptic properties of the liquid are not reduced. Thus, the liquid may be kept essentially at temperature between 0 and 90 degrees C, such as between 0 and 50 degrees C, between 30 and 80 degrees C and between 60 and 90 degrees C. It is presently preferred that the temperature of the liquid is allowed to increase during the treatment with ultrasound, but the temperature of the liquid may also be kept constant, or substantially constant, during the treatment with ultrasound, eg by cooling of the container or tube wherein the liquid is sonicated.

In an interesting embodiment, the sonication is combined with heat treatment of the liquid, e.g. by performing the sonication immediately before, during or immediately after heat treatment (such as pasteurization). This may be done by performing the sonication together with pasteurization in a combined apparatus (the sonication equipment (the ultrasound unit or the sonicator) is integrated with a pasteurizer). Preferably, pasteurization is performed at a temperature of between 60 and 90 degrees C, i.e. 75 degrees C.

In embodiments of the method of the invention the sonication may be carried out as a batch operation, or as a continuous operation, and/or the sonication may carried in a single sonication unit (a flow cell with a sonicator), or in an array of sonication units, connected in a series.

In an interesting embodiment, the sonication is carried out in a flow cell which has a volume of 0.1 to 10 liter. When using a flow cell, the sonication may be performed with a flow rate from 1 to 50000 liter per hour. The flow rate can be increased if two, three, four or more sonication units are connected in a series (array).

In a further embodiment of the present invention, the sonication unit or array is incorporated in a process line, such as a dairy process line. A dairy process line is e.g. the equipment used a dairy for processing the milk into cheese, cheese curd, and/or whey. A dairy process line is e.g. disclosed in US patent 6548089 or in "Dairy Processing Handbook" by Tetra Pak.

Thus, the method of the invention may be carried out in the food industry, e.g. in a dairy or in a winery. In an interesting embodiment, the sonication is carried out in a dairy process line, e.g. prior to or during a pasteurization step, in the cheese vat (prior to, during and/or after removal of the curd), and/or after the outlet where the whey is drained.

In other embodiments, the sonication is carried out at the entrance of the milk or whey to the dairy, and/or the sonication is carried out inside tubes and fittings involved in draining of the whey from the cheese vat, said sonication being performed during or after draining of the whey, and/or the sonication is carried out on the whey, e.g. after the whey has been removed from the cheese vat, and/or the sonication is carried out in the cheese vat, after removal of the curd, and/or the sonication is carried out in the cheese vat, after removal of the curd and the whey, i.e. as a cleaning step.

The bacteriophage may be a lactic acid bacterium phage, e.g. a phage which lyses a bacterial strain of a genus selected from the group consisting of: *Lactococcus, Streptococcus, Lactobacillus, Leuconostoc, Pseudoleuconostoc, Pediococcus, Brevibacterium, Enterococcus, Bifidobacterium* and *Propionibacterium,* and/or a phage which lyses a bacterial strain of species selected from the group consisting of: *Lactococcus lactis, Streptococcus thermophilus, Lactobacillus bulgaricus, Leuconostoc mesenteroides,* and *Bifidobacterium animalis.*

In an presently preferred embodiment of the method of the invention, the sonication is performed at a flow (L/hour) calculated to be in the range from 0.005 * power sonicator (W) / $Log_{10}$ (phages/ml) to 0.20 * power sonicator (W) / $Log_{10}$ (phages/ml) (such as in the range from 0.010 * power sonicator (W) / $Log_{10}$ (phages/ml) to 0.10 * power sonicator (W) / $Log_{10}$ (phages/ml), or in the range from 0.020 * power sonicator (W) / $Log_{10}$ (phages/ml) to 0.080 * power sonicator (W) / $Log_{10}$ (phages/ml)), wherein $Log_{10}$ (phages/ml) is the concentration of the phages in the liquid.

In an interesting embodiment of the method of the present invention, the sonication is performed at an effect (Power in watt) calculated to be in the range from 5 * flow (L/hour) * $Log_{10}$ (phages/ml) to 200 * flow (L/hour) * $Log_{10}$ (phages/ml), (such as in the range from 10 * flow (L/hour) * $Log_{10}$ (phages/ml) to 100 * flow (L/hour) * $Log_{10}$ (phages/ml) or in the range from 15 * flow (L/hour) * $Log_{10}$ (phages/ml) to 60 * flow (L/hour) * $Log_{10}$ (phages/ml)), wherein flow (L/hour) is the desired flow through the flow cell and $Log_{10}$ (phages/ml) is the concentration of the phages in the liquid.

These ranges are based on the calculation of holding time of the liquid in a continuous flow sonicator (flow cell system, sonicator unit):

Our experiments have shown that application of an effect of approx. 1750 W (input to the sonicator) to one liter of phage containing liquid for one minute will inactivate 90% of the phages. The inactivation is approx. logarithmic, i.e. only a $log_{10}$ will survive per minute of sonication.

In accordance with this finding, we assume that the optimal flow though the flow cell for reducing the phage count can be found using the following model:

$$\text{Flow (L/hour)} = 0.034 * \text{power sonicator (W)} / Log_{10} \text{(phages/ml)}$$

Of course the effect of the sonicator unit and/or the flow rate through the flow cell can be varied depending of the count of phages in the liquid, or varied depending on the desired phage count in the liquid after sonication.

Typically, the titer of phages in milk is 1000/ml to 10000/ml, which in case of the sonicator unit uses 20 kW, gives an optimal flow of around 200 L/hour (in the range of 170 L/hour to 227 L/hour). The titer of phages in whey is typically 10000/ml to 100000/ml, which gives an optimal flow of around 150 L/hour (in the range of 136 to 170 L/hour). In the above calculations, the sonicated liquid will theoretically contain 1 phage per mL, which is acceptable for most applications, but it is clear to the skilled person that other flow velocities may be calculated depending on the acceptable phage titer in the sonicated liquid.

Similarly, the optimal power input can be calculated as follows:

$$\text{Power (W)} = 30 * \text{flow (L/hour)} * Log_{10} \text{(phages/ml)}$$

In a second aspect, the present Invention relates to an apparatus (or equipment) for inactivation of bacteriophages in a liquid, wherein said apparatus Is a dairy process line comprising a sonicator or a sonicator unit and a cheese vat (e.g. a container of 100 to 20000 liters, and having an Inlet and an outlet, and optionally an opening for removal of curd (coagulated milk).

In an Interesting embodiment of the apparatus of the invention, a sonicator may be connected to the cheese vat by tubes or pipes (such as tubes/pipes made of steel, metal, plastic, rubber, or glass) and/or a sonicator may placed before and/or after the cheese vat, and/or is integrated in the cheese vat, and/or a sonicator may be connected to the inlet and/or a sonicator Is connected to the outlet.

Other pieces of dairy equipment, such as a pasteurizer or homogenizer, may be connected to the dairy process line. Thus, In an embodiment, the apparatus comprises a sonicator and a pasteurizer, both connected to the inlet; the pasteurizer placed before and/or after the sonicator.

In further embodiments, the apparatus comprises both a sonicator and a pasteurizer connected to the inlet, wherein the sonicator is integrated with the pasteurizer; and/or the apparatus comprises a sonicator unit and a pasteurizer, both connected to the outlet; the pasteurizer placed before or after the sonicator unit; and/or the apparatus comprises both a sonicator unit and a pasteurizer connected to the outlet, wherein the sonicator is Integrated with the pasteurizer; and/or the apparatus comprises a sonicator which is integrated In the cheese vat. For instance, 1, 2, 3, 4 and up to 20 sonicator units may be build-in in the walls (incl top or bottom) of the cheese vat.

A sonicator unit may be connected to the inlet of the cheese vat, and/or a sonicator unit may be connected to the outlet of the cheese vat.

Further pieces of equipment, such as one or more of: a storage tank, an evaporator, a mixing tank, a cream separator, a tank for preparing starter cultures and/or a process tank may be connected to the dairy process line.

In a presently preferred embodiment of the apparatus of the invention the outlet of the sonicator is connected to a storage tank, e.g. to a storage tank which outlet Is connected to the cheese vat, either directly or indirectly via e.g. a pasteurizer.

It should be understood that the invention also comprises an apparatus as described above, wherein the cheese vat and/or dairy process line contains a liquid, such as milk, a milk solution, juice, soy milk or whey.

In a third aspect, the invention relates to the use of a sonicator for inactivation of bacteriophages in a dairy product, such as cheese, cheese curd, fermented milk, milk, whey, whey permeate or cream.

Also disclosed is a dairy product, such as cheese, cheese curd, fermented milk, milk, whey, whey permeate or cream, obtained by a method of the Invention.

Also disclosed is a bacterium selected from the group consisting of: *Lactococcus lactis* strain CHCC2281 (DSM 12087), *Streptococcus thermophilus* strain CHCC9204 (DSM 19243), and mutants/variants of these strains.

Also disclosed Is a bacteriophage selected from the group consisting of The *Lactococcus lactis* bacteriophage CHPC1184 (DSM 24632), the *Streptococcus thermophilus* phage CHPC1057 (DSM 23962), and mutants/variants of these phages.

In a further aspect, the present invention relates a method for treatment of milk, comprising sonication (ultrasound treatment) of the milk, wherein the milk is subjected to ultrasound resulting in an amount of energy applied to the milk in the range of 25 to 400 watt * hour per liter milk.

An interesting embodiment of this aspect is a method for treatment of milk, comprising sonication (ultrasound treatment) of the milk, wherein the milk Is subjected to ultrasound resulting In an amount of energy applied to the milk In the range of 50 to 300 watt * hour per liter milk. Other interesting ranges are: 50 to 300, 75 to 200, 25 to 150, 75 to 150, 100 to 200, and 100 to 150 (watt * hour per liter milk).

In a further aspect, the present invention relates to a method for treatment of milk, comprising sonication of the milk in a flow cell (continuous operation), wherein the sonication Is performed at a flow (L/hour) calculated to be in the range from 0.005 * power sonicator (W) / $\mathrm{Log}_{10}$ (phages/ml) to 0.20 * power sonicator (W) / $\mathrm{Log}_{10}$ (phages/ml) (such as in the range from 0.010 * power sonicator (W) / $\mathrm{Log}_{10}$ (phages/ml) to 0.10 * power sonicator (W) / $\mathrm{Log}_{10}$ (phages/ml), or in the range from 0.020 * power sonicator (W) / $\mathrm{Log}_{10}$ (phages/ml) to 0.080 * power sonicator (W) / $\mathrm{Log}_{10}$ (phages/ml)), wherein $\mathrm{Log}_{10}$ (phages/ml) is the concentration of the phages in the liquid.

In yet an aspect, the present invention relates to a method for treatment of milk, comprising sonication of the milk in a flow cell (continuous operation), wherein the sonication is performed at an effect (Power in watt) calculated to be in the range from 5 * flow (L/hour) * $\mathrm{Log}_{10}$ (phages/ml) to 200 * flow (L/hour) * $\mathrm{Log}_{10}$ (phages/ml), (such as in the range from 10 * flow (L/hour) * $\mathrm{Log}_{10}$ (phages/ml) to 100 * flow (L/hour) * $\mathrm{Log}_{10}$ (phages/ml) or In the range from 15 * flow (L/hour) * $\mathrm{Log}_{10}$ (phages/ml) to 60 * flow (L/hour) * $\mathrm{Log}_{10}$

(phages/ml)), wherein flow (L/hour) is the desired flow through the flow cell and $\mathrm{Log}_{10}$ (phages/ml) is the concentration of the phages in the liquid.

Specific embodiments

Particularly preferred embodiments of the present invention include:

- A method for inactivating bacteriophages in a dairy product comprising sonication of said dairy product, wherein sonication is carried out by treatment of the dairy product with ultrasound at an effect of 1000 to 2000 watt per liter liquid for 1 to 10 minutes, preferably less than 7 minutes, and more preferably less than 5 minutes.

- A method for inactivating bacteriophages in milk or whey comprising sonication of said milk or whey, wherein sonication is carried out by treatment of the milk or whey with ultrasound at an effect of 1000 to 2000 watt per liter liquid for 1 to 10 minutes, preferably less than 7 minutes, and more preferably less than 5 minutes.

- A method for inactivating bacteriophages in a dairy product comprising sonication of said dairy product, wherein sonication is carried out by treatment of the dairy product with ultrasound for 1 to 10 minutes at a frequency in the range of 15000 to 25000 Hz, preferably 18000 to 22000 Hz.

- A method for inactivating bacteriophages in milk or whey comprising sonication of said milk or whey, wherein sonication is carried out by treatment of the milk or whey with ultrasound for 1 to 10 minutes at a frequency in the range of 15000 to 25000 Hz, preferably 18000 to 22000 Hz.

- A method for inactivating bacteriophages in a dairy product comprising sonication of said dairy product, wherein sonication is carried out in a flow cell having a volume of 0.1 to 10 liter with a flow rate of between 1 to 50000 liter per hour, more preferably 100 to 10000 liter per hour, and even more preferably 1000 to 5000 liter per hour.

- A method for inactivating bacteriophages in milk or whey comprising sonication of said milk or whey, wherein sonication is carried out in a flow cell having a volume of 0.1 to 10 liter with a flow rate of between 1 to 50000 liter per hour, more preferably 100 to 10000 liter per hour, and even more preferably 1000 to 5000 liter per hour.

- A method for inactivating bacteriophages in a dairy product comprising sonication of said dairy product, wherein sonication is carried out by treatment of the dairy product with ultrasound at an effect of 1000 to 2000 watt per liter liquid, and wherein said sonication is carried out prior to or simultaneously with heat treatment.

- A method for inactivating bacteriophages in milk or whey comprising sonication of said milk or whey, wherein sonication is carried out by treatment of the milk or whey with ultrasound at an effect of 1000 to 2000 watt per liter liquid, and wherein said sonication is carried out prior to or simultaneously with heat treatment.

**DEFINITIONS**

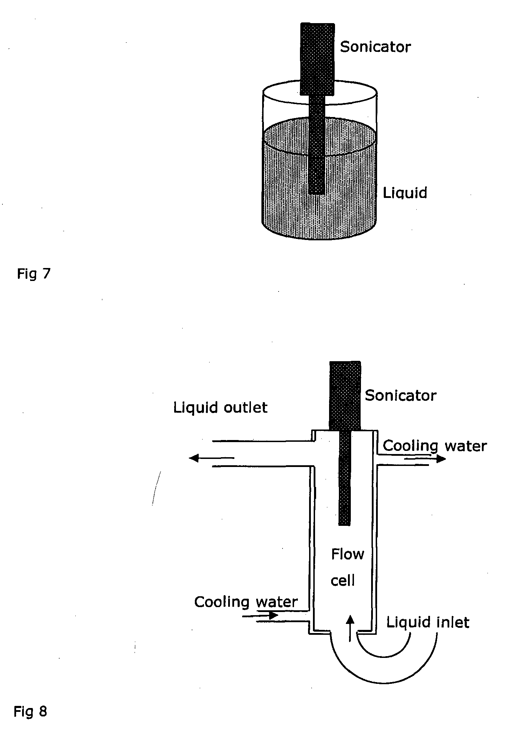

In the present context, the term "sonication", or ultrasound treatment, refers to addition of sound energy at a wave length above 10000 Hz to a liquid or viscous sample. The apparatus emitting the sound energy is a "sonicator". The sonicator (or sonicator unit if the sonicator is attached to a flow cell) is brought in contact with the liquid to be treated. In the present context, the power (calculated in W) and "power sonicator (W)" is the power applied to the liquid (and not the power applied to the sonicator, which might be higher due to loss in the sonicator). In the present context a sonicator may be single unit (cf. figure 7), or an array of sonicator units (cf. figure 8). Sonicators are commercially obtainable, e.g. from Hielscher Ultrasonics GmbH, Warthestr. 21, 14513 Teltow, Germany.

In the present context, the term "liquid" refers to any liquid or viscous sample as e.g. milk, milk solution, whey, water, waste water (e.g. from a dairy), soy milk or juice, e.g. grape juice.

The term "bacteriophage" refers to an enveloped or non-enveloped virus that is capable of infecting bacteria, such as lactic acid bacteria. Infection of lactic acid bacteria by phages can cause fermentation failure in the dairy industry due to impaired growth characteristics or lysis of the infected cells. The method of the invention aims at the "inactivation" of the bacteriophages in a liquid. As used herein, the term "inactivation" means that the bacteriophages treated by sonication are deprived of their ability to infect and/or lyse bacterial cells.

The term "dairy" refers to a location wherein milk, milk solutions or milk fractions (such as whey) is processed. Thus the term includes e.g. cheese factories, yoghurt factories, powder milk factories, whey protein factories.

The term "milk" is to be understood as the lacteal secretion obtained by milking any mammal, such as cows, sheep, goats, buffaloes or camels. In a preferred embodiment, the milk is cow's milk. The term milk also comprises soy milk and other milks of plant origin. Optionally the milk is acidified, e.g. by addition of an acid (such as citric, acetic or lactic acid) or by fermentation, or the milk may be mixed with another ingredient or a liquid, e.g. with water. The milk may be raw or processed, e.g. by filtering, sterilizing, pasteurizing, homogenizing etc, or it may be reconstituted dried milk. An important example is "bovine milk". It is understood that the milk may be acidified, fermented, mixed, treated with a coagulant (e.g. an aspartic protease) or processed before, during and/or after the sonication.

In the present context, the term "milk solution" may be any liquid comprising lactose and protein. Thus, useful milk substrates include, but are not limited to, solutions/suspensions of any milk or milk like products comprising protein, such as whole or low fat milk, skim milk, buttermilk, reconstituted milk powder, condensed milk, milk treated with a protease (e.g. a clotting enzyme, rennet or chymosin), whey, whey permeate, mother liquid from crystallization of lactose, whey protein concentrate, or cream. Obviously, the milk substrate may originate from any milk as defined above. Preferably, at least part of the protein in the milk substrate is proteins naturally occurring in milk, such as casein or whey protein. However, part of the protein may be proteins which are not naturally occurring in milk. Prior to, after and/or during the sonication, the milk substrate may be homogenized and pasteurized according to methods known in the art.

"Homogenizing" as used herein means intensive mixing to obtain a soluble suspension or emulsion. If homogenization is performed prior to fermentation, it may be performed so as to break up the milk fat into smaller sizes so that it no longer separates from the milk. This may be accomplished by forcing the milk at high pressure through small orifices.

"Pasteurizing" as used herein means treatment of the milk substrate to reduce or eliminate the presence of live organisms, such as microorganisms. Preferably, pasteurization is attained by maintaining a specified temperature for a specified period of time. The specified temperature is usually attained by heating. The temperature and duration may be selected in order to kill or inactivate certain bacteria, such as harmful bacteria. A rapid cooling step may follow.

As used herein, the term "lactic acid bacterium" designates a gram-positive, microaerophilic or anaerobic bacterium, which ferments sugars with the production of acids including lactic acid as the predominantly produced acid. The industrially most useful lactic acid bacteria are found within the order "Lactobacillales" which includes *Lactococcus* spp., *Streptococcus* spp., *Lactobacillus* spp., *Leuconostoc* spp., *Pseudoleuconostoc* spp., *Pediococcus* spp., *Brevibacrerium* spp., *Enterococcus* spp. and *Propionibacterium* spp. Additionally, lactic acid producing bacteria belonging to the group of the strict anaerobic bacteria, bifidobacteria, i.e. *Bifidobacterium* spp., are generally included in the group of lactic acid bacteria. These are frequently used as food cultures alone or in combination with other lactic acid bacteria.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising", "having", "including" and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

## EXAMPLES

Example 1:

Sonication of *Lactococcus lactis* phage CHPC1184 in milk, whey and Ringer solution to compare the destruction time in these three media.

*Lactococcus lactis* phages CHPC1184 (DSM 24632) were added to whole milk (pasteurized, non homogenized), whey from cheese fermentation (pH 5,6) and Ringer solution, respectively, in an amount of $10^5$ phages pr. ml. 40 ml samples of the resulting milk, whey and Ringer solution were sonicated for a total of 4 min (Misonix sonicator 3000, power 75 W, end temperature 50°C). Every 30 sec a 200 $\mu$l sample was withdrawn and the number of functional phages counted using the spot test method. In this method 10 $\mu$l samples are spotted on a lawn of the host bacteria (CHCC2281, DSM12087) on a M17-agar plate with 0,5% lactose and 10 mM $CaCl_2$. After incubation over night at 30°C each clearing zone represents one phage. Using this method the detection limit was 100 phages pr. ml.

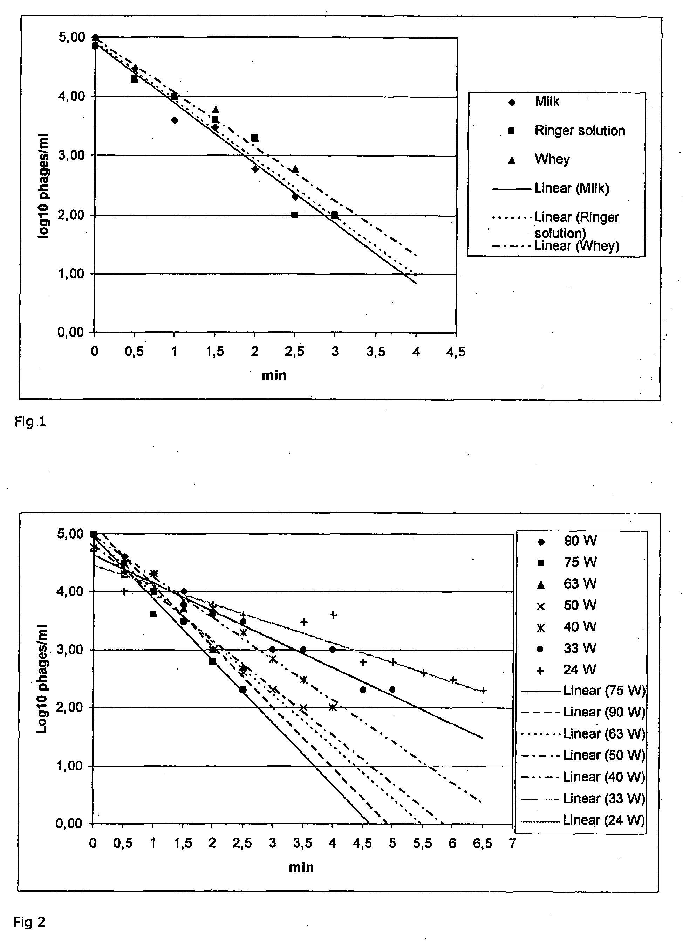

The results showed that the phages were destructed with the same rate in the three media with approx. 1 $\log_{10}$ decimation pr minute (Figure 1).

Example 2:

Sonication of *Lactococcus lactis* phage CHPC1184 in milk at different power settings.

Phages were added to milk as described in example 1. The resulting milk was sonicated with power settings varying from 24-90 W for a total of 7 min (Figure 2). Every 30 sec a 200 $\mu$l sample was withdrawn and the number of functional phages counted as described in example 1.

The results showed inversely proportionality between power and decimation times of phages as clarified in table 1:

Table 1. $\log_{10}$ decimation times of phages in minutes at different power settings.

| Power (W) | $\log_{10}$ decimation time (min) |
|---|---|
| 90 | 0,9 |
| 75 | 0,9 |
| 63 | 1,1 |
| 50 | 1,2 |
| 40 | 1,4 |

(continued)

| Power (W) | $Log_{10}$ decimation time (min) |
|---|---|
| 33 | 2,0 |
| 24 | 3,0 |

Example 3:

Sonication of two different concentrations of *Lactococcus lactis* phage CHPC1184 in milk.

Phages were added to whole milk in an amount of $10^5$ and $10^8$ phages pr. ml, respectively. 40 ml samples of the resulting milk were sonicated for 6 min at 63 W. Every 30 sec a 200 $\mu$l sample was withdrawn and the number of phages counted as described in example 1.

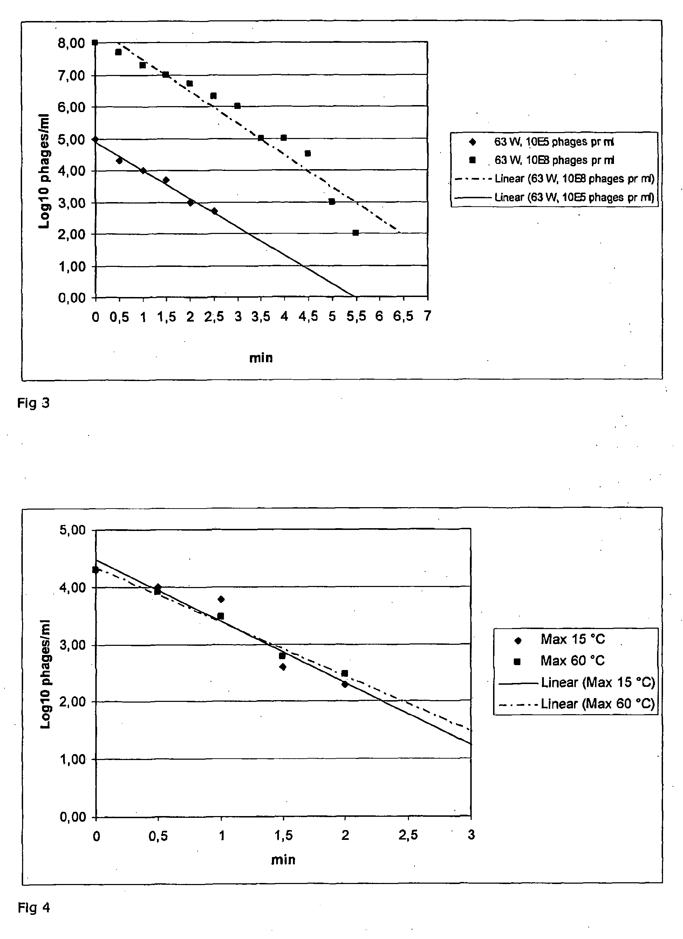

The results showed that phage destruction of $10^8$ phages pr. ml as compared to $10^5$ phages pr. ml resulted in translation of the phage titer/time-line by three log-units (Figure 3). The calculated time used for total phage destruction increased by three minutes from 5.5 min to 8.5 min.

Example 4:

Sonication of *Lactococcus lactis* phage CHPC1184 in milk at two different temperatures.

Phages were added to milk as described in example 1. The resulting milk was sonicated at 63 W as described in example 1 using ice bath as cooling (end temperature 15 °C) or no cooling (end temperature 60 °C).

The results showed no significant difference in phage destruction dependent of the end temperature of the milk (Figure 4).

Example 5:

Sonication of *Lactococcus lactis* phage CHPC1184 and *Streptococcus thermophilus* phage CHPC1057 (DSM 23962), respectively, in whey to compare the destruction kinetics of these two phages.

*Lactococcus lactis* phage CHPC1184 and *Streptococcus thermophilus* phage CHPC1057 were added to whey in a concentration of $10^5$ phages/ml and sonicated as described in example 1. *Streptococcus thermophilus* strain CHCC9204 was used as host strain for CHPC1057.

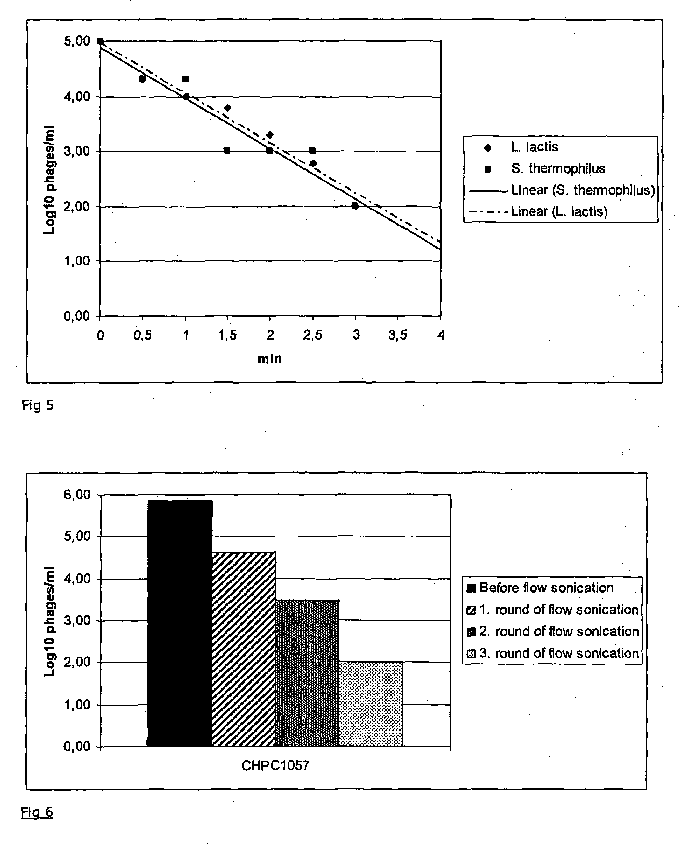

The results showed no difference in destruction kinetics of the two phages (Figure 5). For both phages in whey, the destruction rate was 1 $log_{10}$ decimation pr minute.

Example 6:

Sonication of *Streptococcus thermophilus* phage CHPC1057 in whey using a flow cell system as illustrated in figure 8.

*The* CHPC1057 *phages* were diluted in whey solution to a titer of $10^5$ phages pr. ml. A 250 ml sample of the resulting whey was sonicated using a flow cell compatible with the Misonix sonicator 3000 and a probe tip diameter of ½". Power used was 72 W. The flow cell had a cooling jacket as illustrated in figure 8 and tap water was used as cooling water. The sample was lead through the flow cell with the rate of 10 ml/min using a pump (Masterflex L/S 77200-50). The sonicator was started before the pump. The sample was lead through the flow cell 3 times. Each time a 100 $\mu$l sample was withdrawn and the number of functional phages counted using the spot test method described in example 1. *Streptococcus thermophilus* strain CHCC9204 was used as host strain for CHPC1057.

The results showed that the phage titer of CHCC1057 decreased by nearly 4 log units after 3 rounds of flow cell sonication (Figure 6).

Example 7:

The liquid, such as milk, can be treated by batch sonication, see figure 7. Preferably the volume should not exceed 5 L.

Example 8:

The liquid such as milk can be treated by continuous sonication in a flow cell system, see figure 8. This system may be obtained from Hielscher Ultrasonics and manages from 15L/hr to 50m$^3$/hr depending of the size of the flow cell and power. Sonication generates heat therefore the flow cell is equipped with a cooling jacket.

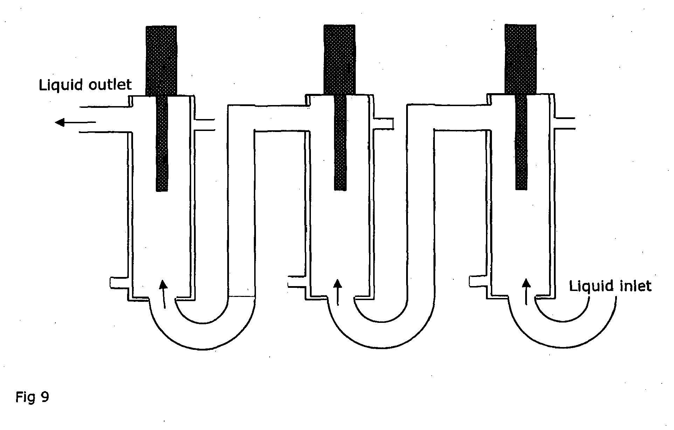

For more efficient sonication it is possible to use sonication flow cells arranged in series as illustrated in figure 9. An appropriate system may be obtained from Hielscher Ultrasonics. Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein. Accordingly, this invention includes all modifications and equivalents of the subject matter recited in the claims appended hereto as permitted by applicable law. Moreover, any combination of the above-described elements in all possible variations thereof is encompassed by the invention unless otherwise indicated herein or otherwise clearly contradicted by context.

## DRAWING

Figure 1 depicts the results of sonication of phages in Milk, whey and Ringer solution. Number of phages (log$_{10}$ phages per ml sample) as a function of sonication time in minutes.

Figure 2 depicts the results of sonication of phages at different power settings. Number of phages (log$_{10}$ phages per ml sample) as a function of sonication time in minutes.

Figure 3 depicts the results of sonication of two different concentrations of phages in milk (10$^5$ and 10$^8$ phages pr. ml). Number of phages (log$_{10}$ phages per ml sample) as a function of sonication time in minutes.

Figure 4 depicts the results of sonication of 10$^5$ phages pr. ml in milk at two different end temperatures. Number of phages (log$_{10}$ phages per ml sample) as a function of sonication time in minutes.

Figure 5 depicts the results of sonication of 10$^5$ *L. lactis* and S. *thermophilus* phages pr. ml in whey. Number of phages (log$_{10}$ phages per ml sample) as a function of sonication time in minutes.

Figure 6 depicts the results of flow sonication of approx. 10$^5$S. *thermophilus* phages (CHPC1057) pr. ml in whey. The whey samples were flow sonicated in a total of 3 rounds. Each bar represents the phage titer after one round of flow sonication. The detection limit was 10$^2$ phages /ml.

Figure 7 depicts equipment for batch sonication of liquid.

Figure 8 depicts equipment for continuous sonication of liquid in a flow cell system. Inlet and outlet can be exchanged, ie the flow through the cell can be reversed.

Figure 9 depicts equipment for sonication of liquid in serial sonication flow cells. Each sonication flow cell unit is as in figure 8.

## DEPOSITS and EXPERT SOLUTION

The *Lactococcus lactis* bacteriophage CHPC1184 was deposited at Deutsche Sammlung von Mikroorganismen und Zellkulturen GmbH, Inhoffenstr. 7B, D-38124 Braunschweig (DSMZ) 03 March 2011 and given the accession number DSM 24632.

Other deposits at DSMZ:

*Lactococcus lactis* strain CHCC2281, Date: 6 Apr. 1998, accession number DSM 12087.
*Streptococcus thermophilus* phage CHPC1057, date: 27 Aug 2010, accession number DSM 23962.
*Streptococcus thermophilus* strain CHCC9204, date: 29 Mar 2007, accession number DSM 19243.

The deposits were made according to the Budapest treaty on the International recognition of the deposit of microorganisms for the purposes of patent procedure.

The Applicant requests that a sample of the deposited microorganism should be made available only to an expert approved by the Applicant.

## REFERENCES

Patent documents: WO92/13068A1, WO93/13674A1, US4086057, US2009/0274768A1. www.hielscher.com

Dairy Processing Handbook (1995), published by Tetra Pak Processing Systems AB, S-22186 Lund, Sweden

**Claims**

1. A method for inactivation of bacteriophages in a liquid, comprising sonication (ultrasound treatment) of the liquid, wherein the liquid is selected from the group consisting of: a dairy product, milk, milk solution, whey, must, and juice.

2. The method of claim 1, wherein the sonication is carried out by treatment of the liquid with ultrasound at a frequency in the range of 10000 to 50000 Hz, such as in the range of 13000 to 34000, 15000 to 25000 Hz or in the range of 18000 to 22000 Hz.

3. The method of any of claims 1-2, wherein the sonication is carried out by treatment of the liquid with ultrasound for 0.1 to 30 minutes, such as for 1.0 to 15, 1.2 to 11, 1.5 to 9, 2.0 to 8, or for 2.5 to 7 minutes.

4. The method of any of claims 1-3, wherein the sonication is carried out by treatment of the liquid with ultrasound at an effect of 100 to 20000 watt per liter liquid, such as 200 to 15000, 300 to 10000, 400 to 5000, or 600 to 2250 watt.

5. The method of any of claims 1-4, wherein the sonication is combined with heat treatment of the liquid, e.g. by performing the sonication immediately before, during or immediately after heat treatment (such as pasteurization).

6. The method of any of claims 1-5, wherein the sonication is carried out in a dairy process line, e.g. prior to or during a pasteurization step, in the cheese vat (prior to, during and/or after removal of the curd), and/or after the outlet where the whey is drained.

7. The method of any of claims 1-6, wherein the sonication is carried out on the whey, e.g. after the whey has been removed from the cheese vat.

8. The method of any of claims 1-6, wherein the sonication is carried out in the cheese vat, after removal of the curd.

9. The method of any of claims 1-8, wherein the bacteriophage is a lactic acid bacterium phage.

10. The method of claim 9, wherein the bacteriophage is a phage which lyses a bacterial strain of a genus selected from the group consisting of: *Lactococcus, Streptococcus, Lactobacillus, Leuconostoc, Pseudoleuconostoc, Pediococcus, Brevibacterium, Enterococcus, Bifidobacterium* and *Propionibacterium.*

11. The method of claim 10, wherein the bacteriophage is a phage which lyses a bacterial strain of a species selected from the group consisting of: *Lactococcus lactis, Streptococcus thermophilus, Lactobacillus bulgaricus, Leuconostoc mesenteroides,* and *Bifidobacterium animalis.*

12. An apparatus for inactivation of bacteriophages in a liquid, wherein said apparatus is a dairy process line comprising a sonicator or a sonicator unit and a cheese vat (e.g. a container of 100 to 20000 liters, and having an inlet and an outlet).

13. The apparatus of claim 12, wherein the sonicator unit is placed before the inlet and/or after the outlet of the cheese vat, and/or the sonicator is integrated in the cheese vat.

14. The apparatus of any of claims 1-13, wherein a pasteurizer Is connected to the dairy process line.

15. The apparatus of any of claims 1-14, which comprises a sonicator unit and a pasteurizer, both connected to the inlet; wherein the pasteurizer is placed before or after the sonicator unit, or wherein the sonicator unit is integrated with the pasteurizer.

16. The apparatus of any of claims 1-14, which comprises a sonicator unit and a pasteurizer, both connected to the outlet; wherein the pasteurizer is placed before or after the sonicator unit, or wherein. the sonicator unit is integrated with the pasteurizer.

17. Use of a sonicator for inactivation of bacteriophages in a dairy product, such as cheese, cheese curd, fermented milk, milk, whey, whey permeate or cream.

**Patentansprüche**

1. Verfahren zur Inaktivierung von Bakteriophagen in einer Flüssigkeit, das die Beschallung (Ultraschallbehandlung) der Flüssigkeit umfasst, wobei die Flüssigkeit ausgewählt ist aus der Gruppe bestehend aus: einem Milchprodukt, Milch, Milchlösung, Molke, Most und Saft.

2. Verfahren nach Anspruch 1, bei dem die Beschallung durch Behandlung der Flüssigkeit mit Ultraschall bei einer Frequenz im Bereich von 10.000 bis 50.000 Hz durchgeführt wird, beispielsweise im Bereich von 13.000 bis 34.000 Hz, 15.000 bis 25.000 Hz oder im Bereich von 18.000 bis 22.000 Hz.

3. Verfahren nach einem der Ansprüche 1-2, bei dem die Beschallung durch Behandlung der Flüssigkeit mit Ultraschall für 0,1 bis 30 Minuten durchgeführt wird, beispielsweise für 1,0 bis 15, 1,2 bis 11, 1,5 bis 9, 2,0 bis 8 oder für 2,5 bis 7 Minuten.

4. Verfahren nach einem der Ansprüche 1-3, bei dem die Beschallung durch Behandlung der Flüssigkeit mit Ultraschall mit einer Wirkung von 100 bis 20.000 Watt pro Liter Flüssigkeit durchgeführt wird, beispielsweise 200 bis 15.000, 300 bis 10.000, 400 bis 5.000 oder 600 bis 2.250 Watt.

5. Verfahren nach einem der Ansprüche 1-4, bei dem die Beschallung mit einer Wärmebehandlung der Flüssigkeit kombiniert wird, z.B. indem die Beschallung unmittelbar vor, während oder unmittelbar nach der Wärmebehandlung (wie z.B. Pasteurisierung) angewendet wird.

6. Verfahren nach einem der Ansprüche 1-5, bei dem die Beschallung in einer Molkerei-Prozesslinie durchgeführt wird, z.B. vor oder während eines Pasteurisierungsschritts, im Käsekessel (vor, während und/oder nach Entfernung des Käsebruchs) und/oder nach dem Auslass, bei dem die Molke abgeschüttet wird.

7. Verfahren nach einem der Ansprüche 1-6, bei dem die Beschallung auf die Molke angewendet wird, z.B. nachdem die Molke aus dem Käsekessel entfernt wurde.

8. Verfahren nach einem der Ansprüche 1-6, bei dem die Beschallung im Käsekessel durchgeführt wird, nach Entfernen des Käsebruchs.

9. Verfahren nach einem der Ansprüche 1-8, bei dem der Bakteriophage ein Phage eines Milchsäurebakteriums ist.

10. Verfahren nach Anspruch 9, bei dem der Bakteriophage ein Phage ist, der einen Bakterienstamm einer Gattung lysiert, die ausgewählt ist aus der Gruppe bestehend aus: *Lactococcus, Streptococcus*, *Lactobacillus, Leuconostoc, Pseudoleuconostoc, Pediococcus, Brevibacterium, Enterococcus, Bifidobacterium* und *Propionibacterium.*

11. Verfahren nach Anspruch 10, bei dem der Bakteriophage ein Phage ist, der einen Bakterienstamm einer Spezies lysiert, die ausgewählt ist aus der Gruppe bestehend aus: *Lactobacillus lactis, Streptococcus thermophilus, Lactobacillus bulgaricus, Leuconostoc mesenteroides* und *Bifidobacterium animalis.*

12. Vorrichtung zur Inaktivierung von Bakteriophagen in einer Flüssigkeit, wobei die Vorrichtung eine Molkerei-Prozesslinie ist, die ein Ultraschallgerät oder eine Ultraschalleinheit und einen Käsekessel (z.B. einen Behälter mit 100 bis 20.000 Litern, der über einen Einlass und einen Auslass verfügt) umfasst.

13. Vorrichtung nach Anspruch 12, wobei die Ultraschalleinheit vor dem Einlass und/oder hinter dem Auslass des Käsekessels angebracht ist und/oder das Ultraschallgerät im Käsekessel integriert ist.

14. Vorrichtung nach einem der Ansprüche 1-13, wobei eine Pasteurisiervorrichtung mit der Molkerei-Prozesslinie verbunden ist.

15. Vorrichtung nach einem der Ansprüche 1-14, die eine Ultraschalleinheit und eine Pasteurisiervorrichtung umfasst, welche beide mit dem Einlass verbunden sind, wobei die Pasteurisiervorrichtung vor oder hinter der Ultraschalleinheit angebracht ist, oder wobei die Ultraschalleinheit in der Pasteurisiervorrichtung integriert ist.

16. Vorrichtung nach einem der Ansprüche 1-14, die eine Ultraschalleinheit und eine Pasteurisiervorrichtung umfasst, welche beide mit dem Auslass verbunden sind, wobei die Pasteurisiervorrichtung vor oder hinter der Ultraschalleinheit angebracht ist, oder wobei die Ultraschalleinheit in der Pasteurisiervorrichtung integriert ist.

17. Verwendung eines Ultraschallgeräts zur Inaktivierung von Bakteriophagen in einem Milchprodukt, wie beispielsweise Käse, Käsebruch, fermentierte Milch, Milch, Molke, Molke-Permeat oder Sahne.

**Revendications**

1. Procédé pour l'inactivation de bactériophages dans un liquide, comprenant la sonication (traitement par ultrasons) du liquide, où le liquide est sélectionné du groupe composé de: un produit laitier, lait, solution de lait, petit-lait, moût et jus.

2. Procédé selon la revendication 1, où la sonication est effectuée par traitement du liquide avec ultrasons à une fréquence de l'ordre de 10000 à 50000 Hz, comme par exemple de l'ordre de 13000 à 34000, 15000 à 25000 Hz ou de l'ordre de 18000 à 22000 Hz.

3. Procédé selon l'une quelconque des revendications 1-2, où la sonication est effectuée par traitement du liquide avec ultrasons pour 0,1 à 30 minutes, comme par exemple 1,0 à 15, 1,2 à 11, 1,5 à 9, 2,0 à 8 ou 2,5 à 7 minutes.

4. Procédé selon l'une quelconque des revendications 1-3, où la sonication est effectuée par traitement du liquide avec ultrasons à un effet de 100 à 20000 watts par litre de liquide, comme par exemple 200 à 15000, 300 à 10000, 400 à 5000, ou 600 à 2250 watts.

5. Procédé selon l'une quelconque des revendications 1-4, où la sonication est combinée avec un traitement thermique du liquide, par exemple en accomplissant la sonication immédiatement avant de, pendant ou immédiatement après le traitement thermique (comme par exemple la pasteurisation).

6. Procédé selon l'une quelconque des revendications 1-5, où la sonication est effectuée dans une chaîne de fabrication laitière, par exemple avant ou pendant une phase de pasteurisation, dans la cuve à fromage (avant, pendant et/ou après l'enlèvement du caillé), et/ou après la sortie où le petit-lait est drainé.

7. Procédé selon l'une quelconque des revendications 1-6, où la sonication est effectuée sur le petit-lait, par exemple après que le petit-lait a été éliminé de la cuve à fromage.

8. Procédé selon l'une quelconque des revendications 1-6, où la sonication est effectuée dans la cuve à fromage, après l'enlèvement du caillé.

9. Procédé selon l'une quelconque des revendications 1-8, où le bactériophage est un phage de bactéries d'acide lactique.

10. Procédé selon la revendication 9, où le bactériophage est un phage qui lyse une souche bactérienne d'un genre sélectionné du groupe composé de: *Lactococcus, Streptococcus, Lactobacillus, Leuconostoc, Pseudoleuconostoc, Pedlococcus, Brevibacterium*, *Enterococcus, Bifidobacterium* et *Propionibacterium*.

11. Procédé selon la revendication 10, où le bactériophage est un phage qui lyse une souche bactérienne d'une espèce sélectionnée du groupe composé de: *Lactococcus lactis, Streptococcus thermophilus, Lactobacillus bulgaricus*, *Leuconostoc mesenteroides,* et *Bifidobacterium animalis.*

12. Dispositif pour l'inactivation de bactériophages dans un liquide, où ledit dispositif est une chaîne de fabrication laitière comprenant un sonicateur ou une unité sonicateur et une cuve à fromage (par exemple un récipient de 100 à 20000 litres, ayant une entrée et une sortie).

13. Dispositif selon la revendication 12, où l'unité sonicateur est placée avant l'entrée et/ou après la sortie de la cuve à fromage, et/ou le sonicateur est intégré dans la cuve à fromage.

14. Dispositif selon l'une quelconque des revendications 1-13, où un pasteurisateur est connecté à la chaîne de fabrication laitière.

**15.** Dispositif selon l'une quelconque des revendications 1-14, comprenant une unité sonicateur et un pasteurisateur, les deux étant connectés avec l'entrée; où le pasteurisateur est placé avant ou après l'unité sonicateur, ou où l'unité sonicateur est intégrée avec le pasteurisateur.

**16.** Dispositif selon l'une quelconque des revendications 1-14, comprenant une unité sonicateur et un pasteurisateur, les deux étant connectés avec la sortie; où le pasteurisateur est placé avant ou après l'unité sonicateur, ou où l'unité sonicateur est intégrée avec le pasteurisateur.

**17.** Utilisation d'un sonicateur pour l'inactivation de bactériophages dans un produit laitier, comme par exemple fromage, fromage de lait caillé, lait fermenté, lait, petit-lait, perméat de petit-lait ou crème.

Fig 1

Fig 2

Fig 3

Fig 4

Fig 5

Fig 6

Fig 7

Fig 8

Fig 9

REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- GB 671922 A
- DE 3903648 A1
- US 6548089 B
- WO 9213068 A1
- WO 9313674 A1
- US 4086057 A
- US 20090274768 A1

**Non-patent literature cited in the description**

- **ATAMER et al.** *Intern Dairy J,* 2010, vol. 20 (3), 163-168
- **TETRA PAK.** Dairy Processing Handbook
- Dairy Processing Handbook. Tetra Pak Processing Systems AB, 1995